# EUROPEAN PATENT SPECIFICATION

(11) **EP 2 586 402 B1**
(45) Date of publication and mention of the grant of the patent: **02.05.2018**
(21) Application number: 12190405.6
(22) Date of filing: 29.10.2012
(51) Int. Cl.: A61F 2/04, A61F 2/82

(54) **Stent with inwardly-directed protrusion**
Stent mit nach innen ragenden Vorsprung
Stent avec projection dirigée vers l'intérieur

(30) Priority: 27.10.2011 US 201161552187 P
(43) Date of publication of application: 01.05.2013
(73) Proprietor: Cook Medical Technologies LLC, Bloomington, IN 47404 (US)
(72) Inventor: Campbell, Triona, Killaloe (IE); Ryan, Michael, Limerick (IE)
(74) Representative: Garratt, Peter Douglas

(56) References cited:
- WO-A2-00/38591
- DE-A1-102005 019 649

## Description

### FIELD

The disclosure relates generally to the field of implantable medical devices. More particularly, the disclosure relates to stents for placement within body vessels, such as the esophagus.

### BACKGROUND

Minimally invasive techniques and instruments for placement of intraluminal medical devices have developed over recent years. A wide variety of treatment devices that utilize minimally invasive technology has been developed and includes stents, stent grafts, occlusion devices, infusion catheters and the like. Stents - frame-like structures placed within a body vessel to provide support to and maintain patency of the vessel - became especially popular with the introduction of coronary stents to the U.S. market in the early 1990s. Since that time, both coronary and peripheral stents have been proven to provide a superior means of maintaining vessel patency, and have become widely accepted in the medical community.

The use of stents has been extended to treatments that target other body vessels. For example, esophageal stents are now widely used to maintain patency of the esophagus from a point within the vessel, such as in the treatment of a stricture that threatens closure of this vessel.

Stenting of the esophagus provides unique challenges not faced by stents intended for other vessels, such as vessels of the vasculature. For example, the muscles that form the esophagus generate peristaltic action - a wavelike series of contractions that forces food through the esophagus and into to the stomach. Stents placed in the esophagus must be able to retain function while resisting migration in this dynamic environment. Furthermore, the lower esophageal sphincter - a muscle near the junction with the stomach - is normally closed to block stomach acid from entering the esophagus. Normally, this muscle only opens during swallowing to allow food to pass into the stomach. After a stent is placed across this muscle, however, the sphincter can remain open in response to the intraluminal support provided by the stent, reducing the ability of the muscle to block acid entry into the esophagus. Over time, acid passage into the esophagus can cause tissue damage, aspiration pneumonia, and other undesirable outcomes.

Reference is directed to WO00/38591 which discloses blood flow tubing with helical flow inducing means and to DE 10 2005 019 649 which discloses a flexible stent with inwardly directed stabilisation members disposed circumferentially.

Thus, a need exists for improved stents.

### BRIEF SUMMARY OF DESCRIBED EMBODIMENTS

Various exemplary stents are described and illustrated herein.

An exemplary stent comprises a main body having a longitudinal axis, a first end, a second end, an outer surface, and an inner surface. The main body defines a lumen extending from the first end to the second end. A protrusion is disposed on and extends inward from the inner surface of the main body. The protrusion extends along a helical path on the inner surface.

In exemplary embodiments, the pitch of the helical path is constant along the axial length of the helical path. In other exemplary embodiments, the pitch of the helical path varies along the axial length of the helical path.

In exemplary embodiments, the depth of the protrusion is constant along the length of the protrusion. In other exemplary embodiments, the depth of the protrusion varies along the length of the protrusion.

In exemplary embodiments, the protrusion has a rectangular cross-sectional shape. In other exemplary embodiments, the protrusion has a curvilinear cross-sectional shape, such as a u-shape.

Additional understanding of these exemplary devices can be obtained with review of the detailed description, below, and the appended drawings.

### BRIEF DESCRIPTION OF THE DRAWINGS

Figure 1 is a perspective view of a first exemplary stent.
Figure 1A is a magnified view of area 1A denoted in Figure 1.
Figure 1B is a magnified view of an alternative structure for area 1A denoted in Figure 1.
Figure 2 is a perspective view of a second exemplary stent.
Figure 3 is a sectional view of the stent illustrated in Figure 2.
Figure 4A is a sectional view of an alternative stent.
Figure 4B is a sectional view of an alternative stent.
Figure 5A is an end view of an alternative stent.
Figure 5B is an end view of an alternative stent.
Figure 5C is an end view of an alternative stent.
Figure 6A is a perspective view of an alternative stent.
Figure 6B is an end view of the stent illustrated in Figure 6A.
Figure 7A is an end view of an alternative stent.
Figure 7B is an end view of an alternative stent.
Figure 7C is an end view of an alternative stent.
Figure 8 is a perspective view, partially broken away, of a third exemplary stent.
Figure 9 is a perspective view of a fourth exemplary stent.
Figure 10 is a perspective view, partially broken away, of a fourth exemplary stent.

### DETAILED DESCRIPTION OF ILLUSTRATED EMBODIMENTS

The following detailed description and the appended drawings describe and illustrate various exemplary stents and methods of treatment. The description and drawings are exemplary in nature and are provided to enable one skilled in the art to make and use one or more exemplary stent and/or to practice one or more exemplary method. They are not intended to limit the scope of the claims in any manner.

As used herein, the term "helical" refers to a path orbiting about a central axis while undergoing translation along the axis. The term does not require any specific or constant pitch, angle, or other parameter.

As used herein, the term "pitch" refers to the width of one complete turn of a protrusion extending along a path on an inner surface of a body, measured parallel to the axis of the body. The term does not require that the path be of any particular configuration.

As used herein, the term "flared end" refers to an end portion of a body having an inner diameter and/or outer diameter that is greater than an inner diameter and/or outer diameter of another portion of the body. The term only requires this relative dimensioning and does not require any particular structure. As such, the term includes various structures that meet the required relative dimensioning, such as conical flares, flanges, stepped flanges, and the like.

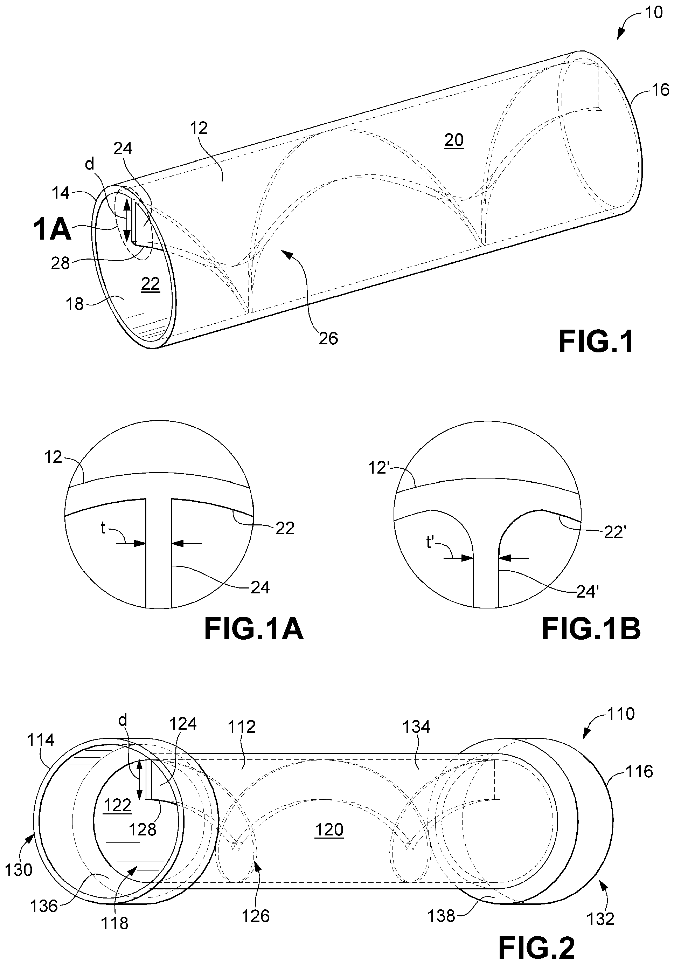

Figure 1 illustrates a first exemplary stent 10. The stent 10 includes a main body 12, a first end 14, a second end 16, and a lumen 18 extending from the first end 14 to the second end 16. The main body 12 has an outer surface 20 and an inner surface 22. A protrusion 24 is disposed on the inner surface 22 and extends along a path 26 on the inner surface 22.

The stent 10 is an expandable stent having radially compressed and radially expanded configurations. The radially compressed configuration is typically used for storage and delivery of the stent 10 to a point of treatment within a body vessel using a delivery device, such as a catheter, as is known in the art. The radially expanded configuration is typically used *in vivo,* following placement of the stent 10 at the point of treatment within the body vessel. In figure 1, the stent 10 is illustrated in the radially expanded configuration.

As illustrated in Figure 1, the path 26 can comprise a helical path that extends from the first end 14 to the second end 16 of the main body. It is noted, though, that the path 26 need not be helical in nature. Indeed, the path 26 can have any suitable configuration, and a skilled artisan will be able to determine an appropriate configuration for a stent according to a particular embodiment based on various considerations, including the body vessel within and point of treatment at the stent is intended to be used. The inventors have determined that a helical path is suitable for stents intended to be implanted within the esophagus at least because such a path provides a suitable structure for allowing food and fluids to pass through the lumen and into the stomach, and for reducing backflow of stomach acid through the stent.

As illustrated in Figure 1, the path 26 can extend along the entire axial length of the main body 12, from the first end 14 to the second end 16. It is noted, though, that the path 26 need not extend along the entire length of the main body 12. Indeed, the path 26 can extend along any suitable length of the main body 12, including a length that is equal to the axial length of the main body 12 or a length that is less than the axial length of the main body 12. A skilled artisan will be able to determine an appropriate axial length - the length of the portion of an axis of the stent along which the protrusion extends - for the path in a stent according to a particular embodiment based on various considerations, including the body vessel within which and the point of treatment at which the stent is intended to be used. The inventors have determined that an axial length that is equal to or substantially equal to the axial length of the main body 12 is suitable for stents intended to be implanted within the esophagus at least because such a length ensures that the backflow-reducing effects of the protrusion 24 are distributed across the entire stent 10. Examples of other lengths considered suitable for the axial length of the protrusion include a length that is less than the axial length of the main body 12. An axial length that is equal to between about 0% and about 100% of the axial length of the main body 12 is considered suitable, particularly for stents intended to be deployed within the esophagus. An axial length that is equal to between about 5% and about 95% of the axial length of the main body 12 is considered suitable, particularly for stents intended to be deployed within the esophagus. An axial length that is equal to between about 15% and about 85% of the axial length of the main body 12 is also considered suitable, particularly for stents intended to be deployed within the esophagus. An axial length that is equal to between about 25% and about 75% of the axial length of the main body 12 is also considered suitable, particularly for stents intended to be deployed within the esophagus. An axial length that is equal to between about 35% and about 65% of the axial length of the main body 12 is also considered suitable, particularly for stents intended to be deployed within the esophagus. An axial length that is equal to between about 45% and about 55% of the axial length of the main body 12 is also considered suitable, particularly for stents intended to be deployed within the esophagus. An axial length that is equal to about 50% of the axial length of the main body 12 is also considered suitable, particularly for stents intended to be deployed within the esophagus.

The path 26 can also have any suitable path length - the actual length of the path as it extends along the inner surface about the axis of the stent. The path 26 can have any suitable path length, including a path length that is less than the axial length of the main body, a path length that is equal to the axial length of the main body, a path length that is substantially equal to the axial length of the main body, and a path length that is greater than the axial length of the main body. A skilled artisan will be able to determine an appropriate path length for the path in a stent according to a particular embodiment based on various considerations, including the body vessel within which and the point of treatment at which the stent is intended to be used. Examples of lengths considered suitable for the path length of the protrusion include a length that is equal to between about 5% and about 1000% of the axial length of the main body 12 is considered suitable, particularly for stents intended to be deployed within the esophagus. A length that is equal to between about 50% and about 500% of the axial length of the main body 12 is also considered suitable, particularly for stents intended to be deployed within the esophagus. A length that is equal to between about 100% and about 250% of the axial length of the main body 12 is also considered suitable, particularly for stents intended to be deployed within the esophagus.

As illustrated in Figure 1, the protrusion 24 extends from the inner surface 22 to a free edge 28 disposed within the lumen 18 defined by the main body 12. At each point along its length, the protrusion 24 has a depth d that represents the dimension of the protrusion 24 extending from its point of interface with the inner surface 22 of the main body 12 to the free edge 28. In the embodiment illustrated in Figure 1, the depth d is generally less than the radius of the lumen defined by the main body 12. It is noted, though, that the depth d can have any suitable dimension, and a skilled artisan will be able to determine an appropriate dimension for a stent according to a particular embodiment based on various considerations, including the body vessel within and point of treatment at the stent is intended to be used. The inventors have determined that a depth d that is less than the radius of the lumen defined by the main body 12 is suitable for stents intended to be implanted within the esophagus at least because such a depth provides a suitable structure for reducing backflow of stomach acid through the stent. Examples of other suitable depth dimensions include a depth that is greater than the radius of the lumen defined by the main body 12 and a depth that is equal to or substantially equal to the radius of the lumen defined by the main body 12. A depth that is equal to between about 25% and about 75% of the radius of the lumen defined by the main body is also considered suitable, particularly for stents intended to be deployed within the esophagus. A depth that is equal to between about 35% and about 65% of the radius of the lumen defined by the main body is also considered suitable, particularly for stents intended to be deployed within the esophagus. A radius that is equal to between about 45% and about 55% of the radius of the lumen defined by the main body is also considered suitable, particularly for stents intended to be deployed within the esophagus. A depth that is equal to about 50% of the radius of the lumen defined by the main body is also considered suitable, particularly for stents intended to be deployed within the esophagus. It is also noted that the depth d need not be constant over the length of the protrusion 24, and indeed the depth d can vary along the length of the protrusion 24. Specific examples of alternative depth dimensions and variable depths are described in detail below.

As illustrated in Figure 1A, the protrusion 24 can contact or join the inner surface 22 of the main body 12 to form an angle. Alternatively, as illustrated in Figure 1B, the protrusion 24 can contact or join the inner surface 22' of the main body 12' to form a curvilinear surface. Also, as illustrated in Figure 1A, the protrusion 24 can have a constant or substantially constant thickness t along its depth d. Alternatively, the protrusion can have a thickness that varies along its length. For example, as illustrated in Figure 1B, the protrusion 24' can have a thickness t' that is relatively narrow at the free edge and relatively wide at the point of interface with the inner surface 22'. In all embodiments, any suitable thickness and/or variation in the thickness of the protrusion can be used, and a skilled artisan will be able to select an appropriate thickness and/or variation in the thickness, and determine whether a variation in the thickness is desirable, based on various considerations, including any desired collapsibility and/or flexibility characteristics of the stent.

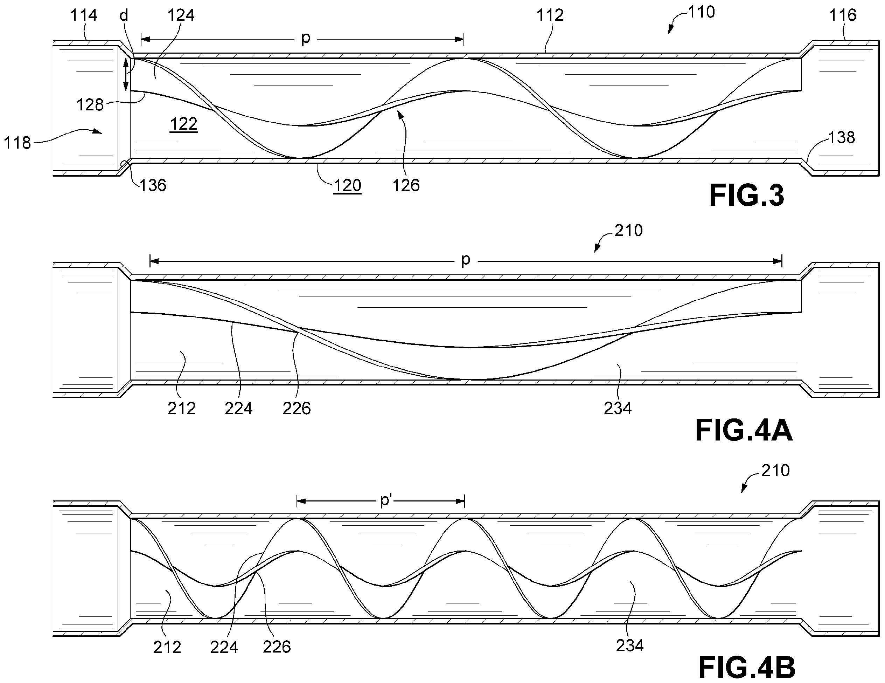

Figures 2 and 3 illustrates a second exemplary stent 110. The stent 110 is similar to the stent 10 described above and illustrated in Figure 1, except as detailed below. Thus, the stent 110 includes a main body 112 having an outer surface 120 and an inner surface 122. A protrusion 124 is disposed on the inner surface 122 and extends along a path 126 on the inner surface 122.

In this embodiment, the main body 112 includes first 130 and second 132 flared ends, and a central portion 134 extending between the flared ends 130, 132. Each of the flared ends 130, 132 has an inner diameter that is greater than an inner diameter of the central portion 134 of the main body 112 when the stent is in the expanded configuration. Also, as illustrated in Figure 2, each flared end 130, 132 can include a transition portion 136, 138 that transitions from the larger inner diameter of the respective flared end 130, 132 to the smaller inner diameter of the central portion 134.

While the stent 110 illustrated in Figures 2 and 3 includes two flared ends 130, 132, it is noted that a stent according to an embodiment could include only a single flared end. A skilled artisan will be able to determine whether one or two flared ends are desirable for a stent according to a particular embodiment based on various considerations, including the body vessel within which and the point of treatment at which the stent is intended to be used. The inventors have determined that inclusion of two flared ends is suitable for stents intended to be implanted within the esophagus at least because such a configuration provides desirable anchoring characteristics. Furthermore, the inventors have determined that the anchoring characteristics provided by such a configuration are particularly well suited for esophageal stents intended to be implanted across the lower esophageal sphincter.

In the embodiment illustrated in Figures 2 and 3, the path 126 extends along the entire axial length of the central portion 134, but does not extend into either of the flared ends 130, 132. It is noted, though, that, as described above, the path 126 can extend along any suitable axial length of the main body 112, including the entire axial length of the main body 112, which includes each of the flared ends 130, 132. Thus, the protrusion 124 can extend into each of the flared ends 130, 132 to any suitable length, including to the respective end 114, 116 of the main body 112. A skilled artisan will be able to determine an appropriate axial length for the path relative to one or more flared ends in a stent according to a particular embodiment based on various considerations, including the body vessel within which and the point of treatment at which the stent is intended to be used. The inventors have determined that an axial length that extends along the entire axial length of the central portion 134 of the main body but that does not extend into either of the flared ends 130, 132 is suitable for stents intended to be implanted within the esophagus at least because such a configuration allows for a greater degree of flexibility in the flared ends 130, 132, which is expected to provide desirable anchoring characteristics while also providing the desired affects of the protrusion 124. Furthermore, the inventors have determined that the anchoring characteristics provided by such a configuration are particularly well suited for esophageal stents intended to be implanted across the lower esophageal sphincter. An axial length that extends along substantially the entire axial length of the central portion 134 of the main body is also considered suitable for stents intended to be implanted within the esophagus. Also, the path can have any suitable path length, as described above, including the exemplary suitable path lengths described above.

Similar to the stent 10 described above and illustrated in Figure 1, the protrusion 124 extends from the inner surface 122 to a free edge 128 disposed within the lumen 118 defined by the main body 112. At each point along its length, the protrusion 124 has a depth *d* that represents the dimension of the protrusion 124 extending from its point of interface with the inner surface 122 of the main body 112 to the free edge 128. In the embodiments described above in which the path 126 extends into the flared ends 130, 132, the depth for the portion or portions of the protrusion 124 disposed within the flared ends 130, 132 can be the same as a depth *d* of the protrusion at a point within the central portion 134 of the main body 112. Alternatively, the depth for the portion or portions of the protrusion 124 disposed within the flared ends 130, 132 can be different than a depth *d* of the protrusion 124 at a point within the central portion 134 of the main body 112. It is noted, though, that the depth of any portion of the protrusion 124 disposed in a flared end 130, 132 can have any suitable dimension, and a skilled artisan will be able to determine an appropriate dimension for a stent according to a particular embodiment based on various considerations, including the body vessel within and point of treatment at the stent is intended to be used. It is also noted that in these embodiments the depth need not be constant over the length of the portion or portions of the protrusion 124 that are disposed in the flared ends 130, 132, and indeed the depth *d* can vary along the length of such portion or portions.

The inventors have determined that various configurations of the pitch of the path, the depth of the protrusion, and the configuration of the protrusion provide desired properties for particular stents. Examples of such configurations are described below and illustrated in the referenced figures. In each of Figures 4A, 4B, 5A, 5B, 5C, 6A, 6B, 7A, 7B, and 7C, the illustrated stent is similar to the stent 110 illustrated in Figures 2 and 3, except where indicated. Thus, the reference numbers used in conjunction with the description of these figures refer to similar elements of the stent 110 illustrated in Figures 2 and 3 with an offset that is a multiple of one hundred. For example, in Figures 4A and 4B, the protrusion is reference by number 224; in Figures 2 and 3 it is referenced by number 124. Reference numbers that lack a corresponding number in Figures 2 and 3 refer to elements and/or properties of the illustrated embodiment that lack a corresponding element and/or property in the embodiment illustrated in Figures 2 and 3.

In all embodiments, the path along with the protrusion extends can have any suitable pitch. A skilled artisan will be able to determine an appropriate pitch for the path in a stent according to a particular embodiment based on various considerations, including a desired number of turns for the protrusion over the length of the path on the inner surface of the main body of the stent. The inventors have determined that any suitable number of turns can be used, including less than one turn, a single turn, and more than one turn. Furthermore, a whole number of turns is considered suitable, as is any number of turns that includes a partial turn, either as less than a single turn or more than a single turn. In some embodiments, the pitch of the path is selected to match a helical pitch of the main body of the stent. This matching of pitches is considered advantageous in some embodiments at least because it is believed to provide desirable collapsibility and flexibility properties for the overall stent. Furthermore, the pitch need not be constant along the entire protrusion as it extends along the path. A varying pitch can be used and may be desirable for stents according to particular embodiments.

In the embodiment illustrated in Figure 4A, the stent 210 includes a path 226 that has a pitch *p* that produces a single turn of the protrusion 224 along the axial length of the central portion of the main body. In the embodiment illustrated in Figure 4B, the stent 210 includes a path 226 that has a pitch *p'* that produces a approximately four (4) turns of the protrusion 224 along the axial length of the central portion of the main body.

The inventors have determined that a pitch that produces one complete turn of the protrusion is suitable for stents intended to be implanted within the esophagus at least because such a configuration is expected to provide the desired impedance to backflow of stomach acid through the stent while conferring minimal additional rigidity onto the stent as a result of inclusion of the protrusion. Furthermore, the inventors have determined that such a configuration is particularly well suited for esophageal stents intended to be implanted across the lower esophageal sphincter. A pitch that produces a greater number of turns of the protrusion can be used if an increased impedance to backflow of stomach acid is desired. While inclusion of such a pitch is expected to confer additional rigidity onto the stent, the benefit provided by such a pitch may outweigh this effect in certain situations. Examples of suitable numbers of turns for the protrusion along the stent include less than one turn (*e.g.,* 0.25, 0.5, and 0.75 turns), 1, 2, 3, 4, 5, 6, 7, 8, 9, 10, 11, and 12 turns. Even higher numbers of turns can be used if appropriate.

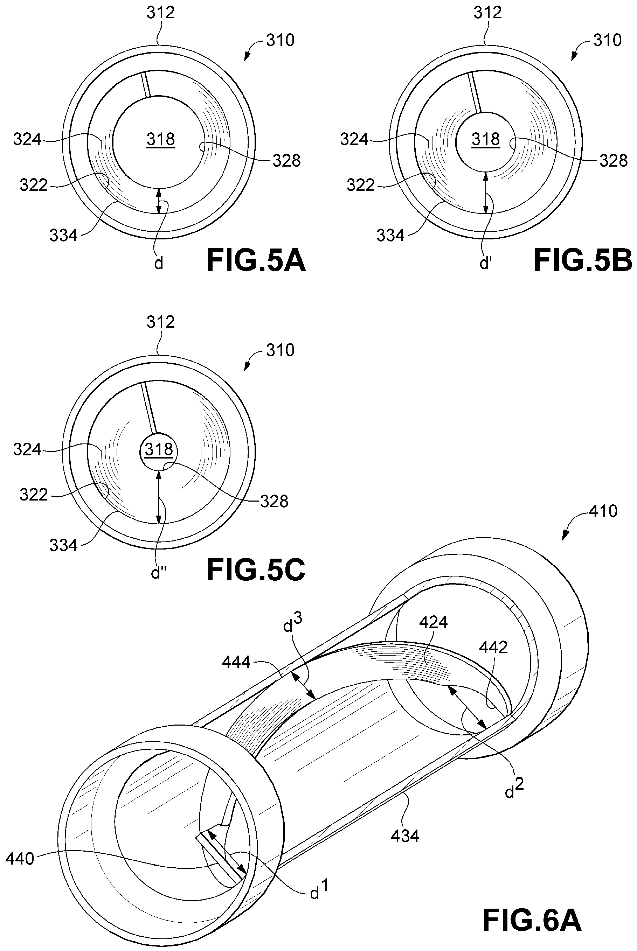

In all embodiments, the protrusion can have any suitable depth. A skilled artisan will be able to determine an appropriate depth for the protrusion in a stent according to a particular embodiment based on various considerations, including a desired degree to which the stent impedes backflow of fluid, such as stomach acid, through the stent and the resulting impact on the overall flexibility of the stent. Figures 5A, 5B and 5C illustrate a stent 310 with various alternative depths. In Figure 5A, the protrusion 324 has a depth *d* that is less than the radius of the lumen 318 defined by the central portion 334 of the main body 312. In Figure 5B, the protrusion 324 has a depth *d'* that about equal to the radius of the lumen 318 defined by the central portion 334 of the main body 312. In Figure 5C, the protrusion 324 has a depth *d"* that is greater than the radius of the lumen 318 defined by the central portion 334 of the main body 312. The inventors have determined that a depth between about 0.1 and 1.5 times the radius of the lumen defined by the main body is suitable. A depth between about 0.3 and 1 times the radius of the lumen defined by the main body is also considered suitable. A depth between about 0.5 and 0.75 times the radius of the lumen defined by the main body is also considered suitable.

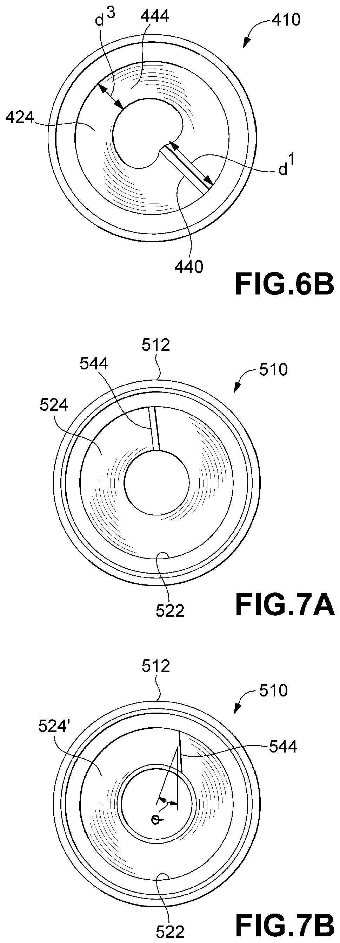

As described above, the depth need not be constant along the entire protrusion as it extends along the path. A varying depth can be used and may be desirable for stents according to particular embodiments. For example, the stent 410 illustrated in Figures 6A and 6B include a protrusion 424 that has a depth that varies as the protrusion extends along a helical path 426 along the inner surface of the central portion 434 of the main body 412. In Figure 6A, a portion of the main body 412 has been removed to more clearly illustrate the protrusion 424 and its varying depth.

At a first end 440 of the protrusion 424, the protrusion 424 has a first depth *d¹.* At a second end 442 of the protrusion 424, the protrusion 424 has a second depth *d².* At a midpoint 444 of the protrusion 424, the protrusion 424 has a third depth *d³.* In this embodiment, *d¹* is equal to *d²* and *d³* is less than both *d¹* and *d¹.* Furthermore, *d³* is equal to about ½ of each of *d¹* and *d².* The depth of the protrusion gradually diminishes from *d¹* at the first end 440, to *d³* at the midpoint 444 of the protrusion 424, and gradually increases from *d³* at the midpoint 444 of the protrusion 424 to *d²* at the second end 442. As best illustrated in Figure 6B, this arrangement provides a central opening 446 for each turn of the protrusion that is offset from the central longitudinal axis of the stent 410. It is noted that, while the illustrated embodiment *d³* is positioned at the midpoint 444, it can be positioned at any point along the protrusion 424.

This arrangement of different depths is exemplary in nature, and any suitable arrangement can be used, including the inverse arrangement in which two smaller depths are placed at the ends of the protrusion and a larger depth is placed at a point in between the ends, an arrangement in which the depth gradually reduces from a relatively large depth at one end of the protrusion to a relatively small depth at the second end of the protrusion, and an arrangement in which the depth gradually increases from a relatively small depth at one end of the protrusion to a relatively large depth at the second end of the protrusion. A skilled artisan will be able to determine an appropriate arrangement of varying depths of the protrusion in a stent according to a particular embodiment based on various considerations, including a desired degree to which the stent impedes backflow of fluid, such as stomach acid, through the stent and the resulting impact on the overall flexibility of the stent.

In all embodiments, the protrusion can have any suitable cross-sectional shape. Furthermore, the protrusion be disposed on the inner surface of the main body in any suitable arrangement. A skilled artisan will be able to determine an appropriate cross-sectional shape and arrangement relative to the inner surface of the main body in a stent according to a particular embodiment based on various considerations, including a desired degree to which the stent impedes backflow of fluid, such as stomach acid, through the stent and the resulting impact on the overall flexibility of the stent.

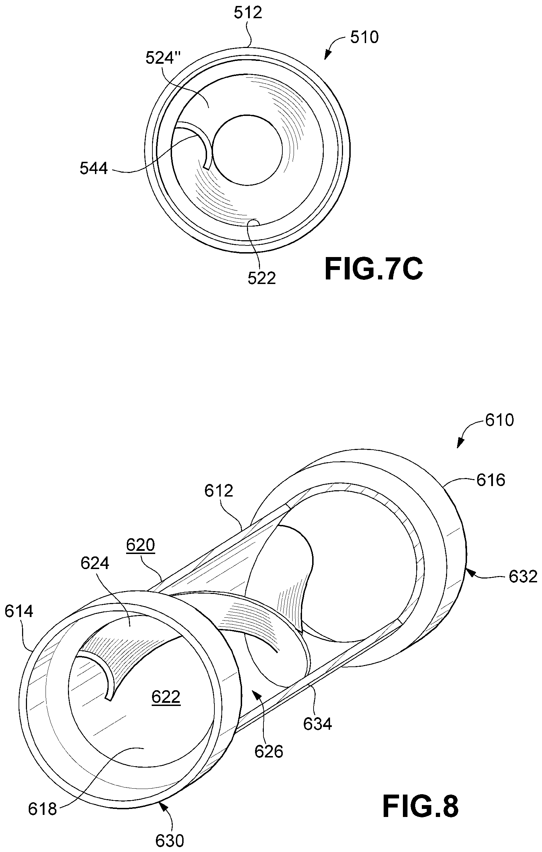

Figures 7A, 7B and 7C illustrate a stent 510 with various exemplary protrusions. In Figure 7A, the protrusion 524 has a rectangular cross-sectional shape and is disposed on the inner surface 522 of the main body 512 such that the first end 440 of the protrusion 524 extends from an interface with the inner surface 522 toward the longitudinal axis of the main body 512 on a plane that contains the longitudinal axis of the main body 512.

In Figure 7B, the protrusion 524' has a rectangular cross-sectional shape and is disposed on the inner surface 522 of the main body 512 such that the first end 540 of the protrusion 524' extends from an interface with the inner surface 522 on a plane that does not contain the longitudinal axis of the main body 512. In this embodiment, the first end 540 of the protrusion 524' lies on a plane that intersects a plane containing the radius *r* extending from the interface of the first end 540 with the inner surface 522 at angle θ. In embodiments that include this arrangement of the protrusion 524' relative to the inner surface of the main body, angle θ can comprise any suitable angle, including an acute angle, as illustrated in Figure 7B, an obtuse angle, and a right angle (illustrated in Figure 7A).

In Figure 7C, the protrusion 524" has a curvilinear cross-sectional shape. Any suitable curvilinear shape can be used, including the u-shaped configuration illustrated in the figure. The inclusion of a protrusion having a curvilinear cross-sectional shape may be advantageous as such a configuration may enhance the ability of food and fluids to flow through the stent in one direction while still providing the desired impedance to backflow of fluid in the reverse direction.

Figure 8 illustrates a third exemplary stent 610. The stent 610 includes a main body 612, a first end 614, a second end 616, and a lumen 618 extending from the first end 614 to the second end 616. The main body 612 has an outer surface 620 (partially removed in the figure) and an inner surface 622. A protrusion 624 is disposed on the inner surface 622 and extends along a helical path 626 on the inner surface 622.

The main body includes first 630 and second 632 flared ends, and a central portion 634 extending between the first 630 and second 632 flared ends. In the illustrated embodiment, the protrusion 624 extends along the entire axial length of the central portion 634, but does not extend into either of the first 632 or second 634 flared ends. The helical path 626 has a pitch that produces a single complete turn of the protrusion 624 as it extends along the axial length of the central portion 634 of the main body 612. In the illustrated embodiment, the protrusion 624 has a curvilinear, u-shaped cross-sectional shape.

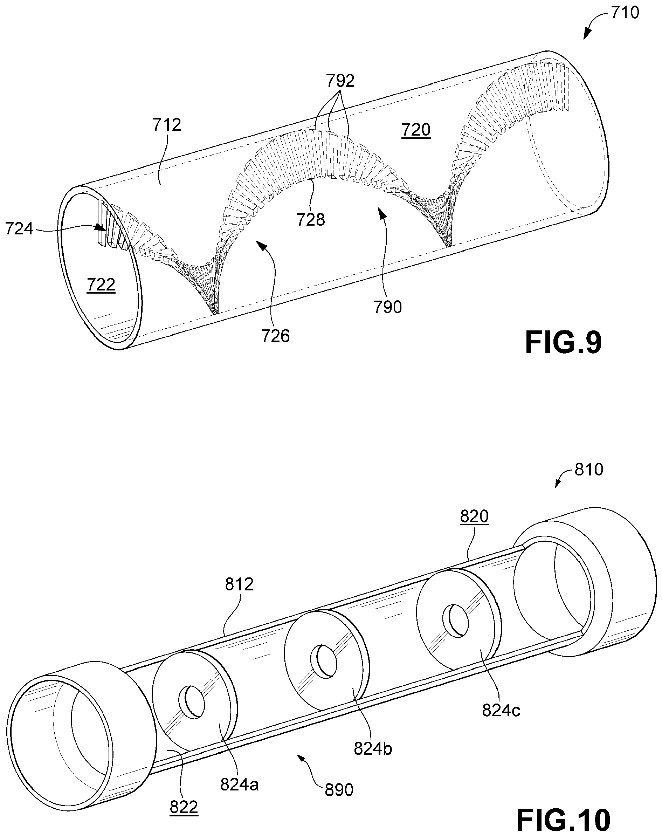

Figure 9 illustrates a fourth exemplary stent 710. The stent 710 is similar to the stent 10 described above and illustrated in Figure 1, except as detailed below. Thus, the stent 710 includes a main body 712 having an outer surface 720 and an inner surface 722. A protrusion 724 is disposed on the inner surface 722 and extends along a path 726 on the inner surface 722.

In this embodiment, the protrusion 724 comprises a series 790 of independent projections 792 extending from the inner surface 722. Each of the projections 722 is independent of all other projections 722 in the series such that a space is disposed between the projection 722 and its neighbor(s). Alternatively, a series of projections can be formed in which the projections are independent of each other along the free edge 728 of the protrusion 724, but that are joined together at the point of interface with the inner surface 722. Such series of projections can be used as an alternative to a single protrusion in any given embodiment. Indeed, in all embodiments, a single protrusion, two protrusions, or a series of protrusions can be used.

Figure 10 illustrates a fifth exemplary stent 810. The stent 810 is similar to the stent 110 described above and illustrated in Figures 2 and 3, except as detailed below. Thus, the stent 810 includes a main body 812 having an outer surface 820 and an inner surface 822.

In this embodiment, a series 890 of protrusions 824a, 824b, and 824c is disposed on the inner surface 822. Each protrusion 824a, 824b, 824c is independent of all other protrusions in the series 890 and extends along a path on the inner surface 822 that is distinct from the path along which the other protrusions extend. In the illustrated embodiment, each of the protrusions 824a, 824b, and 824c comprises a ring-shaped member with a central opening. It is noted, however, that any suitable shape can be used in a stent according to a particular embodiment. Also, while the illustrated embodiment includes three protrusions 824a, 824b, and 824c, any suitable number of protrusions can be used in a stent according to a particular embodiment, and a skilled artisan will be able to select an appropriate number of protrusions based on various considerations, including the length and any desired flexibility of the stent.

Ring-shaped protrusions can be positioned at any point along the length of the stent 810. While the illustrated stent 810 includes ring-shaped protrusions 824a, 824b, 824c positioned along the main body 812 and not within the flared ends, it is noted that one or more ring-shaped protrusions can be disposed within one or both flared end of a stent according to a particular embodiment. In these embodiments, the protrusions can be positioned at any point within the flared end, including at the respective stent end or at a point between the respective stent end and the interface between the flared end and the main body.

In another exemplary embodiment, one or more ring-shaped protrusions are included, as in the embodiment illustrated in Figure 10, and one or more of these protrusions comprises a series of independent projections extending from the inner surface of the main body of the stent, as in the embodiment illustrated in Figure 9.

In all embodiments, the stent and its components can be formed of any suitable material, including presently known and later-developed materials considered suitable for use in long-term *in vivo* medical device implants. A skilled artisan will be able to select appropriate material or materials for a stent according to a particular embodiment based on various considerations, including the nature of the vessel within which the stent is intended to be implanted. Examples of suitable materials include, but are not limited to, stainless steel, nitinol, nickel-cobalt-chromium alloys, nickel-cobalt-chromium-molybdenum alloys, polymeric materials, such as silicone-based materials, polyurethanes, and other polymeric materials, and other biocompatible materials. Nickel-cobalt-chromium-molybdenum alloys, such as MP35N (Carpenter Technology, Wyomissing, PA; MP35N is a registered trademark of SPS Technologies, Inc.), are considered particularly advantageous at least because of the relatively high tensile strength provided by these materials.

In all embodiments, the stent can be self-expandable in nature, in which the stent transitions from a radially compressed configuration to a radially expanded configuration without an input of force following the removal of a constraining force, or can require the input of a radially-outward directed force to affect expansion. Thus, in all embodiments, the stent can comprise a self-expandable stent, a balloon expandable stent, or any other suitable configuration.

For embodiments in which the stent is intended to be implanted in the esophagus of a patient, the inventors have determined that a self-expandable stent formed of a single polymeric material is considered advantageous at least because such materials allow for formation and/or manufacturing of the stent as a unitary member, such as by conventional molding and other techniques.

In all embodiments, the stent and its components can be formed from a single material or multiple materials. For example, a stent according to an embodiment can be formed by molding an appropriate polymeric or other material into the desired configuration such that the main body, with or without flared ends, and the protrusion are integrally formed with each other and of the same material. Alternatively, different materials can be used. For example, a protrusion formed of one material can be attached to a main body formed of another material to form a composite stent.

Furthermore, in all embodiments, the stent can be coated with one or more suitable polymeric or other materials, as is known in the art. For example, stents can be sprayed, dipped rolled, or otherwise exposed to a polymeric material in a manner that produces a desired coating on the stent, either on a portion of a surface, on an entire surface, or on all surfaces of the stent. Additionally, in all embodiments, a sleeve can be placed around the stent as is known in the art, such as a polymeric sleeve.

Also alternatively, the stent can include one or more wire members braided together to form a mesh, such as for the main body. In these embodiments, a separate protrusion can be attached to the inner surface of the main body using suitable attachment techniques and/or components, such as welding, adhering with an adhesive, attaching with mechanical attachment members, and any other suitable technique and/or components.

The foregoing detailed description refers to exemplary occlusion devices and includes the best mode for practicing the invention. The description and the appended drawings illustrating the described devices are intended only to provide examples and not to limit the scope of the claims in any manner.

There have been described stents with inwardly-directed protrusions. An exemplary stent has a main body having a longitudinal axis, a first end, a second end, an outer surface, and an inner surface. The main body defines a lumen extending from the first end to the second end. A protrusion is disposed on and extends inward from the inner surface of the main body. The protrusion extends along a path on the inner surface and has a pitch and a depth.

## Claims

1. An esophageal stent (10), comprising:
a main body (12) having a longitudinal axis, a first end (14), a second end (16), an outer surface (20), an inner surface (22), and defining a lumen (18) extending from the first end to the second end; and
a protrusion (24) disposed on and extending inward from the inner surface relative to the longitudinal axis along a helical path on the inner surface,
**characterised in that** the protrusion has a depth (d) extending from a free edge to the inner surface of the main body which is at least about 25% of the radius of said lumen.

2. The stent of claim 1, wherein the helical path has an axial length and a constant pitch along the axial length.

3. The stent of claim 1, wherein the helical path has an axial length and a varying pitch along the axial length.

4. The stent of any one of the preceding claims, wherein the protrusion has a free edge disposed in the lumen of the main body and a depth extending from the free edge to inner surface of the main body; and
wherein the depth is constant over the length of the protrusion.

5. The stent of any one of the preceding claims, wherein the depth varies over the length of the protrusion.

6. The stent of claim 5, wherein the protrusion has a first depth at a first end, a second depth at a second end, and a third depth at a point between the first and second ends; and
wherein the first depth is approximately equal to the second depth and the third depth is alternatively less than or greater than each of the first depth and the second depth.

7. The stent of any one of the preceding claims, wherein the protrusion has a curvilinear cross-sectional shape.

8. The stent of claim 7, wherein the protrusion has a u-shaped cross-sectional shape.

9. The stent of any one of the preceding claims, wherein the protrusion is integrally formed with the main body.

10. The stent of claim 9, wherein said stent is formed of a polymeric material.

11. The stent of any one of Claims 1 to 8, wherein the protrusion comprises a separate member attached to the main body.

12. The stent of any one of the preceding claims, wherein the main body includes first and second flared ends and a central portion disposed between the first and second flared ends.

13. The stent of claim 12, wherein the protrusion extends along a part or the entirety of an axial length of the central portion of the main body but does not extend into either of the first and second flared ends.

14. The stent of claim 12, wherein the protrusion extends along an axial length of the central portion of the main body and into one or both of the first and second flared ends.

15. The stent of any one of the preceding claims, wherein said stent comprises a self-expandable stent.

## Patentansprüche

1. Speiseröhren-Stent (10), umfassend:
einen Hauptkörper (12), der eine Längsachse, ein erstes Ende (14), ein zweites Ende (16), eine Außenfläche (20) und eine Innenfläche (22) aufweist und ein Lumen (18) definiert, das sich vom ersten Ende zum zweiten Ende erstreckt; und
einen Vorsprung (24), der auf der Innenfläche angeordnet ist und sich von dieser im Verhältnis zur Längsachse entlang eines spiralförmigen Wegs auf der Innenfläche nach innen erstreckt,
**dadurch gekennzeichnet, dass** der Vorsprung eine Tiefe (d) aufweist, die sich von einem freien Rand zur Innenfläche des Hauptkörpers erstreckt, die mindestens ungefähr 25% des Radius des Lumens beträgt.

2. Stent nach Anspruch 1, wobei der spiralförmige Weg eine axiale Länge und eine konstante Steigung entlang der axialen Länge aufweist.

3. Stent nach Anspruch 1, wobei der spiralförmige Weg eine axiale Länge und eine variierende Steigung entlang der axialen Länge aufweist.

4. Stent nach einem der vorhergehenden Ansprüche, wobei der Vorsprung einen freien Rand, der im Lumen des Hauptkörpers angeordnet ist, und eine Tiefe aufweist, die sich vom freien Rand zur Innenfläche des Hauptkörpers erstreckt; und
wobei die Tiefe über die Länge des Vorsprungs konstant ist.

5. Stent nach einem der vorhergehenden Ansprüche, wobei die Tiefe über die Länge des Vorsprungs variiert.

6. Stent nach Anspruch 5, wobei der Vorsprung eine erste Tiefe an einem ersten Ende, eine zweite Tiefe an einem zweiten Ende und eine dritte Tiefe an einem Punkt zwischen dem ersten Ende und dem zweiten Ende aufweist; und
wobei die erste Tiefe ungefähr der zweiten Tiefe gleicht und die dritte Tiefe alternativ kleiner oder größer als jede der ersten Tiefe und der zweiten Tiefe ist.

7. Stent nach einem der vorhergehenden Ansprüche, wobei der Vorsprung eine krummlinige Querschnittsgestalt aufweist.

8. Stent nach Anspruch 7, wobei der Vorsprung eine U-förmige Querschnittsgestalt aufweist.

9. Stent nach einem der vorhergehenden Ansprüche, wobei der Vorsprung einstückig mit dem Hauptkörper ausgebildet ist.

10. Stent nach Anspruch 9, wobei der Stent aus einem Polymermaterial geformt ist.

11. Stent nach einem der Ansprüche 1 bis 8, wobei der Vorsprung ein separates Element umfasst, das am Hauptkörper befestigt ist.

12. Stent nach einem der vorhergehenden Ansprüche, wobei der Hauptkörper erste und zweite aufgeweitete Enden und einen zwischen den ersten und zweiten aufgeweiteten Enden angeordneten Mittelabschnitt umfasst.

13. Stent nach Anspruch 12, wobei sich der Vorsprung entlang eines Teils oder entlang der Gesamtheit einer axialen Länge des Mittelabschnitts des Hauptkörpers erstreckt, sich aber nicht in eines der ersten und zweiten aufgeweiteten Enden erstreckt.

14. Stent nach Anspruch 12, wobei sich der Vorsprung entlang einer axialen Länge des Mittelabschnitts des Hauptkörpers und in eines oder beide der ersten und zweiten aufgeweiteten Enden erstreckt.

15. Stent nach einem der vorhergehenden Ansprüche, wobei der Stent einen selbst-expandierenden Stent umfasst.

## Revendications

1. Endoprothèse oesophagienne (10), comprenant :
un corps principal (12) ayant un axe longitudinal, une première extrémité (14), une seconde extrémité (16), une surface externe (20), une surface interne (22), et délimitant une lumière (18) s'étendant depuis la première extrémité vers la seconde extrémité ; et
une projection (24) disposée sur la surface interne et s'étendant vers l'intérieur depuis celle-ci par rapport à l'axe longitudinal le long d'un trajet hélicoïdal sur la surface interne,
**caractérisée en ce que** la projection a une profondeur (d) s'étendant depuis un bord libre vers la surface interne du corps principal qui représente au moins 25 % environ du rayon de ladite lumière.

2. Endoprothèse selon la revendication 1, dans laquelle le trajet hélicoïdal a une longueur axiale et un pas constant le long de la longueur axiale.

3. Endoprothèse selon la revendication 1, dans laquelle le trajet hélicoïdal a une longueur axiale et un pas variable le long de la longueur axiale.

4. Endoprothèse selon l'une quelconque des revendications précédentes, dans laquelle la projection a un bord libre disposé dans la lumière du corps principal et une profondeur s'étendant depuis le bord libre vers la surface interne du corps principal ; et
dans laquelle la profondeur est constante sur la longueur de la projection.

5. Endoprothèse selon l'une quelconque des revendications précédentes, dans laquelle la profondeur varie sur la longueur de la projection.

6. Endoprothèse selon la revendication 5, dans laquelle la projection a une première profondeur au niveau d'une première extrémité, une deuxième profondeur au niveau d'une seconde extrémité, et une troisième profondeur au niveau d'un point entre les première et seconde extrémités ; et
dans laquelle la première profondeur est approximativement égale à la deuxième profondeur et la troisième profondeur est alternativement inférieure ou supérieure à chacune de la première profondeur et de la deuxième profondeur.

7. Endoprothèse selon l'une quelconque des revendications précédentes, dans laquelle la projection a une coupe transversale curviligne.

8. Endoprothèse selon la revendication 7, dans laquelle la projection a une coupe transversale en U.

9. Endoprothèse selon l'une quelconque des revendications précédentes, dans laquelle la projection fait partie intégrante du corps principal.

10. Endoprothèse selon la revendication 9, ladite endoprothèse étant constituée d'un matériau polymère.

11. Endoprothèse selon l'une quelconque des revendications 1 à 8, dans laquelle la projection comprend un élément distinct fixé au corps principal.

12. Endoprothèse selon l'une quelconque des revendications précédentes, dans laquelle le corps principal comprend des première et seconde extrémités évasées et une partie centrale disposée entre les première et seconde extrémités évasées.

13. Endoprothèse selon la revendication 12, dans laquelle la projection s'étend le long d'une partie ou de la totalité d'une longueur axiale de la partie centrale du corps principal, mais ne s'étend pas dans l'une ou l'autre des première et seconde extrémités évasées.

14. Endoprothèse selon la revendication 12, dans laquelle la projection s'étend le long d'une longueur axiale de la partie centrale du corps principal et dans l'une des première et seconde extrémités évasées ou les deux.

15. Endoprothèse selon l'une quelconque des revendications précédentes, ladite endoprothèse comprenant une endoprothèse autodéployable.
